(19) Europäisches Patentamt
European Patent Office
Office européen des brevets

(11) Publication number: **0 608 419 A1**

(12) # EUROPEAN PATENT APPLICATION published in accordance with Art. 158(3) EPC

(21) Application number: **92920034.3**

(22) Date of filing: **16.09.92**

(86) International application number: **PCT/JP92/01177**

(87) International publication number: **WO 93/05777 (01.04.93 93/09)**

(51) Int. Cl.5: **A61K 31/335**, A61K 9/08

(30) Priority: **18.09.91 JP 268228/91**
**30.07.92 JP 203600/92**

(43) Date of publication of application: **03.08.94 Bulletin 94/31**

(84) Designated Contracting States: **AT BE CH DE DK ES FR GB GR IE IT LI LU NL SE**

(71) Applicant: **FUJISAWA PHARMACEUTICAL CO., LTD.**
**4-7, Doshomachi 3-chome**
**Chuo-ku**
**Osaka-shi Osaka 541(JP)**

(72) Inventor: **SHIMOJO, Fumio, 2-2-13, Dai-wahigashi**
**Kawanishi-shi**
**Hyogo 666-01(JP)**
Inventor: **SAWAI, Seiji, 5-3-104, Yashiro-cho**
**Takarazuka-shi**
**Hyogo 665(JP)**
Inventor: **HORI, Sadao, 3-22-13-503, Minamiotsuka**
**Toshima-ku**
**Tokyo 170(JP)**
Inventor: **KOSEKI, Yoshiyuki, 2-1-45-505, Ikenohata**
**Taito-ku**
**Tokyo 110(JP)**
Inventor: **TERANO, Hiroshi, 2600-3, Manabe**
**Tsuchiura-shi**
**Ibaraki 300(JP)**
Inventor: **OKUHARA, Masakuni, 2-14-10, Umezono**
**Tsukuba-shi**
**Ibaraki 305(JP)**

(74) Representative: **Türk, Gille, Hrabal, Leifert**
**Brucknerstrasse 20**
**D-40593 Düsseldorf (DE)**

(54) **TRANSOCULAR PREPARATION.**

(57) A transocular preparation containing an oxaspirooctane derivative represented by general formula (I) or a salt thereof as an active ingredient and a pharmaceutically acceptable carrier, wherein $R^1$ represents carbamoyl, etc., $R^2$ represents lower alkoxy, etc., and $R^3$ represents a group of formula (II), etc.

EP 0 608 419 A1

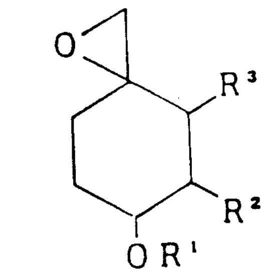

( I )

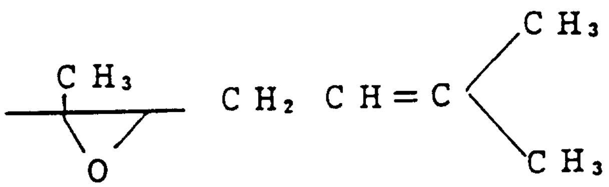

(II)

## TECHNICAL FIELD

The present invention relates to a novel transocular pharmaceutical preparation. More particularly, it relates to a transocular pharmaceutical preparation comprising oxaspirooctane derivatives, represented by the formula (I) described below, or salts thereof as an active ingredient and pharmaceutically acceptable carrier(s).

## BACKGROUND ART

Oxaspirooctane derivatives (I), salts thereof, processes for preparation thereof and their inhibitory action against vascularization have been known as disclosed art, for example, in Japanese Patent Application Laid-open No. HEI 2-85272.

Since the oxaspirooctane derivatives (I) have the inhibitory action against vascularization, they have been expected to be used in the treatment for diseases, to which vascularization is related mostly, such as hyperplastic diabetic retinopathy and senile degeneration of macula lutea. However, when the oxaspiroooctane derivatives (I) are administered systemically to a patient orally or by an injection, the patient are to be suffered from a side effect in which the oxaspirooctane derivatives (I) suppress the vascularization of the normal region that is essential to the physical growth and maintenance of the patient, as well as the vascularization of the lesioned region.

The present inventors have carried out extensive studies for the purpose of overcoming the said side effect. As a result, notwithstanding the technical common knowledge that drugs are generally hard to be absorbed into posterior eye tissue at a high concentration by transocular administration, the inventors have found that, when the oxaspirooctane derivatives (I) are administered in a form of a transocular pharmaceutical preparation, the oxaspirooctane derivatives (I) can be transferred rapidly at a high concentration to posterior eye tissue such as retina, moreover a concentration ratio of the oxaspirooctane derivatives (I) in eye tissue against in blood is much higher than the concentration ratio in case of subcutaneous administration, and the other. Then, the inventors have done further intensive studies, which lead to accomplishment of the present invention.

## DISCLOSURE OF THE INVENTION

The transocular pharmaceutical preparation (i.e. pharmaceutical preparation to be administered through eye) of the present invention comprises oxaspirooctane derivatives (I) represented by the general formula:

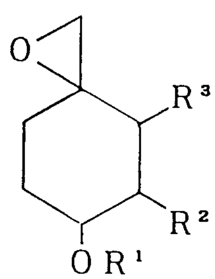

(I)

wherein

$R^1$ is carbamoyl; lower alkylcarbamoyl; hydroxy(lower)alkylcarbamoyl; lower alkoxy(lower)alkylcarbamoyl; lower alkylthio(lower)alkylcarbamoyl; lower alkoxycarbonyl(lower)alkylcarbamoyl; lower alkylcarbamoyloxy-(lower)alkylcarbamoyl di(lower)alkylcarbamoyl; N-[hydroxy(lower)alkyl](lower)alkylcarbamoyl; N-[hydroxy-(lower)alkyl](lower)alkylcarbamoyloxy(lower) alkylcarbamoyl; lower alkylcarbamoyloxy(lower)alkenoyl; N-[heteroocyclic carbonyloxy(lower)alkyl](lower)alkylcarbamoyl; cyclo (lower)alkylcarbamoyl; arylcarbamoyl; haloarylcarbamoyl; protected carbamoyl; lower alkylthiocarbamoyl; heterocyclic carbamoyl; ar(lower)-alkenoyl; lower alkoxycarbonyl; heterocyclic carbonyl which may contain lower alkyl, hydroxy, hydroxy-(lower)alkyl, lower alkoxy(lower)alkyl or lower alkoxycarbonyl; lower alkyl, carboxy(lower)alkyl; protected carboxy(lower)alkyl; ar(lower)alkyl which may contain halogen or lower alkoxy; heterocyclic(lower)alkyl; lower alkylcarbamoyl(lower)alkyl; hydroxy(lower)alkenoyl; acyloxy(lower)alkenoyl or diacyloxy(lower)-alkenoyl;

$R^2$ is lower alkoxy; and

$R^3$ is represented by the following formula:

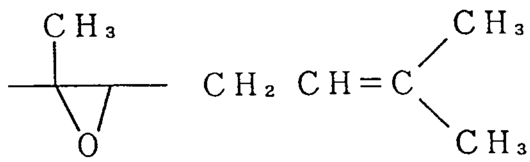

the following general formula:

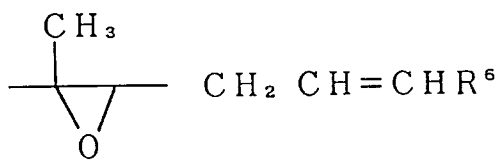

(wherein $R^6$ is protected carboxy), the following general formula:

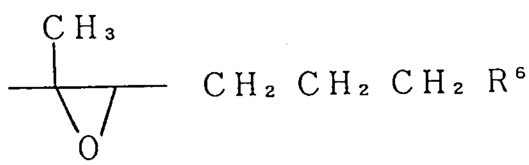

(wherein $R^6$ is the same as defined above), the following formula:

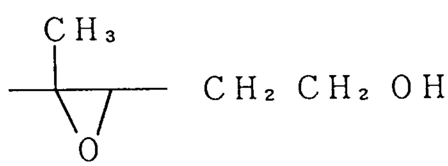

the following general formula:

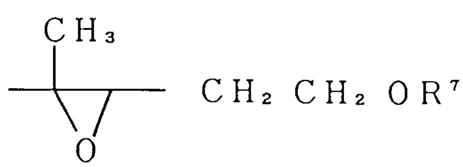

(wherein $R^7$ is protected carboxy(lower)alkyl, or ar(lower)alkyl which may contain halogen), or the following general formula:

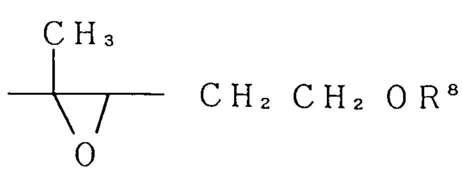

(wherein $R^8$ is acyl) or salts thereof, and pharmaceutically acceptable carrier(s).

BEST MODE FOR CARRYING OUT THE INVENTION

In the oxaspirooctane derivatives (I) as an active ingredient of the present transocular pharmaceutical preparation, the various definitions about the substituents are explained in the followings.

The term "lower" is intended to mean 1 to 6 carbon atoms, unless otherwise indicated.

Suitable "lower alkyl" in the terms "lower alkylcarbamoyl", "hydroxy(lower)alkylcarbamoyl", "lower alkoxy(lower)alkylcarbamoyl", "lower alkylthio(lower)alkylcarbamoyl", "lower alkoxycarbonyl(lower)-alkylcarbamoyl", "lower alkylcarbamoyl(lower) alkylcarbamoyl", "di(lower)alkylcarbamoyl", "ar(lower)alkyl", "N-[hydroxy(lower)alkyl](lower)alkylcarbamoyl", "N-[hydroxy(lower)alkyl](lower)alkylcarbamoyloxy(lower)-alkylcarbamoyl" "lower alkylcarbamoyloxy(lower)alkenoyl", "lower alkylthiocarbamoyl", "lower alkyl", "hydroxy(lower)alkyl", "lower alkoxy(lower)alkyl", "carboxy(lower)alkyl", "protected carboxy(lower)alkyl", "heterocyclic(lower)alkyl" and "lower alkylcarbamoyl(lower)alkyl" may include methyl, ethyl, propyl, isopropyl, butyl, isobutyl, t-butyl, pentyl, hexyl, and the like.

Suitable "lower alkoxy" in the terms "lower alkoxy(lower)alkylcarbamoyl", "lower alkoxycarbonyl(lower)-alkylcarbamoyl", "lower alkoxycarbonyl" and "lower alkoxy(lower)alkyl" may include methoxy, ethoxy, propoxy, butoxy, pentyloxy, hexyloxy, and the like.

Suitable "lower alkylthio" in the term "lower alkylthio(lower)alkylcarbamoyl" may include methylthio, ethylthio, propylthio, butylthio, pentylthio, hexylthio, and the like.

Suitable "lower alkenoyl" in the terms "lower alkylcarbamoyloxy(lower)alkenoyl", "hydroxy(lower)-alkenoyl", "acyloxy(lower)alkenoyl", "diacyloxy(lower)alkenoyl" and "ar(lower)alkenoyl" may include $C_3$-$C_6$ alkenoyl such as acryloyl and crotonoyl.

Suitable "cyclo(lower)alkylcarbamoyl" is "cyclo($C_3$-$C_7$)alkylcarbamoyl", including cyclopropylcarbamoyl, cyclobutylcarbamoyl, cyclopentylcarbamoyl, cyclohexylcarbamoyl, cycloheptylcarbamoyl, and the like.

Suitable "aryl" in the terms "arylcarbamoyl", "haloarylcarbamoyl", "ar(lower)alkyl" and "ar(lower)-alkenoyl" may include phenyl, tolyl, xylyl, naphthyl, and the like.

Suitable halogen in the terms "haloaryl" and "halogen" may include chlorine, bromide, iodine and fluorine.

Suitable "heterocyclic" in the terms "N-[heterocyclic carbonyloxy(lower)alkyl](lower)alkylcarbamoyl", "heterocyclic carbonyl", "heterocyclic carbamoyl" and "heterocyclic(lower) alkyl" may include N-containing heteromonocyclic (e.g. pyridyl, pyrrolidyl, piperidyl, piperazinyl, 2-oxopyrrolidyl, etc.), N,O-containing heteromonocyclic (e.g. molpholinyl, etc.), N,S-containing heteromonocyclic (e.g. thiomorpholinyl, etc.), benzene-condensed N-containing heterocyclic (e.g., quinolyl, etc.), and the like.

Suitable "protected carbamoyl" may include carbamoyl protected by a conventional carbamoyl-protective such as halo(lower)alkanoyl (e.g. trichloroacetyl, dichloroacetyl, monochloroacetyl, etc.), and the like.

Suitable "protected carboxyl" in the terms "protected carboxy(lower)alkyl" and "protected carboxyl may include esterified carboxyl such as lower alkoxycarbonyl (e.g. methoxycarbonyl, ethoxycarbonyl, propoxycarbonyl, isopropoxycarbonyl, butoxycarbonyl, pentyloxycarbonyl, hexyloxycarbonyl, etc.), and the like.

Suitable "acyl" in the terms "acyloxy(lower)alkenoyl" and "diacyloxy(lower)alkenoyl" may include lower alkanoyl (e.g. formyl, acetyl, propionyl, etc.), aroyl (e.g. benzoyl, etc.), lower alkanesulfonyl (e.g. methanesulfonyl, ethanesulfonyl, etc.), and the like.

The salts of oxaspirooctane derivatives (I) may include conventional salts which are pharmaceutically acceptable.

The dosage form of the transocular pharmaceutical preparation of the present invention is not limited particularly, however aqueous solution, non-aqueous solution, suspension and ophthalmic ointment are known as typical examples. In addition, ophthalmic insertion agent, which is filled with an active ingredient to be discharged continuously, can also be exemplified in the present invention.

The pharmaceutically acceptable carriers used in the transocular pharmaceutical preparation of the present invention may include solution or suspension such as water or olive oil, binders such as carboxymethyl cellulose and hydroxypropylmethyl cellulose, isotonic agents such as sodium chloride, buffering agents such as boric acid, sodium hydrogenphosphate and sodium dihydrogenphosphate, preservatives such as benzalkonium chloride, ointment bases such as "Vaseline" (Trademark), polyethylene glycol and hydrocarbon gel (which is a mixture of liquid paraffin and polyethylene resin), resins such as ethylene vinylacetate and polylactic acid.

Among the transocular pharmaceutical preparation of the present invention, preparations of an aqueous solution type, for example, can be prepared by adding powdery oxaspirooctane derivatives (I) or salts thereof into a suitable buffering agent-containing aqueous solution, if required, the resultant solution can be filtered. It is needless to say that auxiliary substances and surfactants can be used simultaneously according to kinds of oxaspirooctane derivatives (I).

As for ophthalmic ointment, the pharmaceutical preparations by the kneading method or by the melting method can be preferably exemplified, particularly the melting method is recommended in the present invention, and in those method "Vaselin" (Trademark), liquid paraffin, lanolin, mixtures thereof, or each of said component or said mixtures with thermoplastic resins such as polyethylene resins are used as a base. In a preparation of the ophthalmic ointments by the melting method, the oxaspirooctane derivatives (I) or salts thereof are mixed with the base exemplified above, particularly hydrocarbon gel composed of liquid paraffin and polyethylene resin, and heated over kneading, when they are melted and mixed in the base, they are cooled rapidly to separate them finely in the base. Since the ophthalmic ointments by the melting method contains an active ingredient dispersed as microcrystallines, they are recommended preferably because of their excellent transitional property into eye tissue. In this specification, the term "excellent transitional property" of an active ingredient means easiness of transition into various eye tissues, or a rise in transition concentration in various eye tissues in a case having higher content in ophthalmic ointment, and furthermore, involves expectation of prolonged transitional property of an active ingredient.

The optimum dosage of oxaspirooctane derivatives (I) or salts thereof, which are active ingredients of the transocular pharmaceutical preparation of the present invention, depends on various factors such as kinds of diseases, conditions of a patient, and so on. However, in general, preferred dosage of oxaspirooctane derivatives (I) or salts thereof is suitably selected within the range of 0.01 to 10 mg/day. An optimum concentration of the active ingredient in the transocular pharmaceutical preparations is suitably selected within the range of 0.01 to 50% (w/w) according to the dosage form of the present invention.

The pharmaceutical preparation of the present invention may be administered to a patient in several times a day separately or continuously in consideration of conditions of the patient.

## EXAMPLE

The present invention is illustrated in more detail according to the following examples.

### Example 1

(1) Test compound:

6-Methylcarbamoyloxy-5-methoxy-4-[2-methyl-3-(3-methyl -2-butenyl)oxiranyl]-1-oxaspiro[2.5]octane

(2) Test preparations:

(a) Pharmaceutical preparation for ophthalmic solution

The test compound described above was dissolved in isotonic borate buffer solution (pH 8) (see Manual of The Japanese Pharmacopoeia XI, Rule of Pharmaceutical Preparations, No. 18: Ophthalmic Solutions) so that a concentration of the compound became 2 mg/ml and the resultant solution was filtered using "Milipore filter" (Trademark) to give a pharmaceutical preparation for ophthalmic solution for transocular administration. The resultant preparation was filled in an instillator.

(b) Pharmaceutical preparation for subcutaneous administration

The test compound described above was dissolved in propylene glycol so that the concentration of the compound became 8.8 mg/ml and the resultant solution was filtered using "Milipore filter" (Trademark) to give a pharmaceutical preparation for subcutaneous administration. The resultant preparation was packed in an osmolal mini-pump (ALzet, model 2ML1, discharge amount: 0.284 ml/day, a one-week discharge type).

(3) Test animals:

c The experiment was carried out using JW (clean) strain female rabbits (body weight: 2.3 to 2.6 kg), each test group containing three rabbits. In this experiment, the rabbits were allowed to feed themselves and

drink water freely both before and after each administration.

(4) Test method:

In case of transocular administration of ophthalmic solution, the rabbits were fixed on the cangue fixer and applied to each eye with one drop (about 0.05 ml) of the above ophthalmic solution using an instillator. The administration was repeated five times at 5-minute intervals. After the final administration, the rabbits were brought back to their cage. In these administrations, total dosage was 1 mg/rabbit. At 30 minutes after the final administration, blood was collected from the ear vein of the rabbits. Immediately, the rabbits were anesthetized by injection of "Nembutal" (Trademark) into the ear vein to bleed to death by cutting off their head and then the eyeballs were taken out therefrom.

On the other hand, in case of continuous subcutaneous administration, after the rabbits were anesthetized by injection of "Nembutal" (Trademark, 0.5 to 0.6 ml/kg i.v.), they were clipped their back side hair and then were incised their clipped parts about 3 cm long with a surgical knife. The clamp was inserted from the incised parts to make a pocket for subcutaneous administration, into which the osmolal mini-pump filled with above preparation for subcutaneous administration (17.6 mg/2 ml) was implanted. Subsequently, the incised parts of the rabbits were sutured with silk thread, and then the rabbits were brought back to their cage. In this case, total dosage was 2.5 mg/rabbit/day. The day when the mini-pump was implanted was defined as "Day 0". On Day 5, the rabbits were anesthetized by injection of "Nembutal" (Trademark) into the ear vein to bleed to death, and then the eyeballs were taken out therefrom. In this experiment, blood was collected for measuring a concentration of the test compound in blood on each Day 1, 4 and 5.

The eyeballs taken out were washed with physiological saline solution under ice-cooling, and subsequently divided into various parts (i.e. cornea, aqueous humor in anterior chamber of eye, iris, lens, vitreum, retinochoroid and sclera). After measuring the weight of each part, phosphate buffer solution (pH 7.4) was added thereto and then the parts were homogenized using a glass-made "Potter-type homogenizer" (Trademark). The resultant was immediately extracted with ethyl acetate and dried to give a sample for measuring a concentration of the test compound in eye tissue.

As for the blood (whole blood), phosphate buffer solution (pH 7.4) was added thereto, and then the blood was extracted with ethyl acetate and dried to give a sample for measuring a concentration of the test compound in blood.

The determination of a concentration of the test compound in each sample was carried out by the two-step Enzyme-linked immunosorbent assay (EIA) using purified polyclonal antibody against the test compound. The preparation method of the polyclonal antibody and ELISA were carried out according to the methods described in "Transplantation Proceeding, 14(5), 23-29 (1987). The results are shown in Tables 1 and 2 below.

Table 1

Transitional property of the test compound into eye tissue by transocular administration of ophthalmic solution to rabbit

(measurement: 30 min. after administration; using three rabbits; tissue: prepared by treating both left and right eyeballs respectively (n = 6); n.d.: 0.05 ng or less /tissue)

| Tissue | Mean concentration in tissue ± S.E. (ng/g or ng/ml) |
|---|---|
| Cornea | 1.56 ± 0.73 |
| Aqueous humor in anterior chamber of eye | 140.64 ± 59.72 |
| Iris | n.d. |
| Lens | 12.45 ± 2.11 |
| Vitreum | 9.99 ± 2.37 |
| Retinochoroid | 40.21 ± 9.40 |
| Sclera | 0.78 ± 0.78 |
| Whole blood (n = 3) | 19.60 ± 4.22 |

Table 2

Transitional property of the test compound into eye tissue measured on Day 5 after continuous subcutaneous administration (2.5 mg/rabbit/day) by subcutaneous implantation of a mini-pump into rabbit

(using three rabbits; tissue: prepared by treating both left and right eyeballs respectively (n = 6); n.d.: 0.05 ng/tissue or less)

| Tissue | Mean concentration in tissue ± S.E. (ng/g or ng/ml) |
|---|---|
| Cornea | 2.87 ± 0.52 |
| Aqueous humor in anterior chamber of eye | 0.79 ± 0.62 |
| Iris | n.d. |
| Lens | 0.20 ± 0.08 |
| Vitreum | 1.19 ± 0.23 |
| Retinochoroid | 0.60 ± 0.26 |
| Sclera | 1.06 ± 0.27 |
| Whole blood, Day 1 (n=3) | 30.58 ± 6.13 |
| Whole blood, Day 4 (n=3) | 13.72 ± 9.74 |
| Whole blood, Day 5 (n=3) | 22.51 ± 4.12 |

Apparently from the above tables, the transitional property of the test compound into eye tissue was highly excellent in case of transocular administration for ophthalmic solutions compared with continuous subcutaneous administration and it was found that the test compound was easy to transfer into eye tissue, particularly retinochoroid, at much higher concentration.

Example 2

Both eyes of five rabbits (Dutch) were used for this experiment. Into the vitreum of the rabbits, 0.2 ml of sterilized air was injected. One or two weeks later, when an exfoliation at the posterior vitreum was confirmed, 80 µg of cupric chloride pellet which was wrapped with "Elvax" (Trademark) was transplanted into inferior cavities of the posterior vitreum. At the same time as transplantation, 1 mg of aminoadipic acid dissolved in 0.2 ml of physiological saline solution was injected into the vitreum, by which vascularization in the eyes (i.e. in the vitreum) was induced. Twenty-four hours later, isotonic phosphate buffer solution (pH 8, 2 mg/ml) which contained the same test compound used in Example 1 was administered drop by drop to each rabbit. The same administration was repeated twice a day for consecutive 12 days. After the final administration on the 12th day, the eyeballs were taken out therefrom and observed histologically to find whether the vascularization was suppressed or not. As a result, it was found that intraocular vascularization was suppressed thoroughly in 42.9% of the eyeballs, and the vascularization was suppressed partially or thoroughly in 85.7% of the eyeballs.

Example 3

(1) Test compound:

The test compound in Example 1 was used for this experiment.

(2) Test preparation:

The above test compound was dispersed in hydrocarbon gel, which was used as ointment base, so that a concentration of the compound became 10%. The hydrocarbon gel used in this experiment was "Plastibase" (Trademark, a mixture of 95 % of liquid paraffin and 5 % of polyethylene resin) produced by Taisho Seiyaku Kabushiki Kaisha.

The test compound in hydrocarbon gel was heated and melted in oil bath at 150 °C for about five min. to give a clear solution. The resultant solution was rapidly cooled with ice under stirring to give a suspension-type ophthalmic ointment in which the test compound was finely dispersed. The resultant ophthalmic ointment was heated with stirring at a temperature (between 85 and 95 °C), where only "Plastibase" (Trademark) was melted and fluidized without melting the finely dispersed test compound, and then filled in an instillator. The instillator was cooled immediately in order to prevent the sedimentation of the test compound.

(3) Transitional property of the test compound into each eye tissue in vivo:

The experiment was carried out using the same strain of rabbits used in Example 1 (body weight; 2.2 to 2.8 kg), each test group containing three to eight rabbits. The rabbits were allowed to feed themselves and drink water freely both before and after administration of the test preparation.

The rabbits were fixed on the canguer fixer and the ophthalmic ointment was administered to each lower eyelid conjunctival sac of both eyes at a single dose of 50 µl. After the administration, the rabbits were fixed on the canguer fixer and blood was collected from the ear vein to measure a concentration of the test compound in blood. The rabbits were anesthetized by injection of "Nembutal" (Trademark, 0.5 to 0.6 ml/kg i.v.) into the ear vein to bleed to death by cutting off their head and then the eyeballs were taken out therefrom. The eyeballs of both left and right eyes were treated respectively as different specimens.

The eyeballs taken out were washed with physiological saline solution over cooling with ice, and divided into each part such as aqueous humor in anterior chamber of eye, iris (containing ciliary body), vitreum and retinochoroid. After measuring the weight of each part, phosphate buffer solution (pH 7.4) was added thereto, and then the parts were homogenized by means of "Biotron" (Trademark). The resultant was extracted immediately with ethyl acetate and dried to give a sample for EIA in eye tissue.

On the other hand, phosphate buffer solution (pH 7.4) was added to blood (whole blood) just after blood collecting, and then the blood was extracted with ethyl acetate and dried to give a sample for EIA in blood.

A concentration of the test compound in each eye tissue, when 10%-ophthalmic ointment was administered to rabbits at a single dose of 50 µl, is shown in Table 3. As a reference, results in case of transocular administration by 0.2%-ophthalmic solution are also shown in the same table. According to the results, the concentration of the test compound in eye tissue reached $C_{max}$ value at 30 min. after administration of the ophthalmic ointment and it was found that transitional property of the test compound

into eye tissue was quite good. As for aqueous humor in anterior chamber of eye and vitreum, transitional property of the test compound into eye tissue was more excellent in the ophthalmic ointment than in the ophthalmic solution, which suggests that the ophthalmic ointment is more effective than ophthalmic solution from the standpoint of drug effect, because the dosage at a single administration can be largely increased in the ophthalmic ointment.

Table 3

| Transitional property of the test compound into eye tissue measured after transocular administration to rabbit | | | | | |
|---|---|---|---|---|---|
| Pharmaceutical preparation | Concentration in eye tissue (ng/g or ng/ml, mean value) (measurement: 0.5 hour after administration) | | | | |
| | Aqueous humor in anterior chamber of eye | Iris | Vitreum | Retinochoroid | Blood |
| 10%-ophthalmic ointment | 5258.0 | 27.2 | 46.5 | 88.8 | 15.5 |
| 0.2%-ophthalmic solution | 516.2 | 73.7 | 17.2 | 31.9 | 12.6 |
| (ophthalmic solution: total dosage: 50 ul, administration: 5 times at 5-min.intervals) | | | | | |

## INDUSTRIAL APPLICABILITY

As shown in the above examples, the transocular pharmaceutical preparation of the present invention is extremely useful for prevention and treatment of diseases such as hyperplastic diabetic retinopathy in which vascularization at retina may cause hemorrhage in eyes thereafter result in blindness, and senile degeneration of macula lutea which features mainly disciform lesion caused by subretinal vascularization.

## Claims

**1.** A transocular pharmaceutical preparation comprising oxaspirooctane derivatives (I) represented by the general formula:

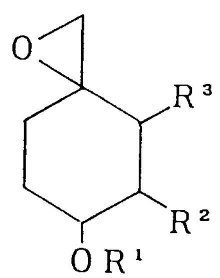

(I)

wherein

$R^1$ is carbamoyl; lower alkylcarbamoyl; hydroxy(lower)alkylcarbamoyl; lower alkoxy(lower)-alkylcarbamoyl; lower alkylthio(lower)alkylcarbamoyl; lower alkoxycarbonyl(lower)alkylcarbamoyl; lower alkylcarbamoyloxy(lower)alkylcarbamoyl; di(lower)alkylcarbamoyl; N-[hydroxy(lower)alkyl](lower)-alkylcarbamoyl; N-[hydroxy(lower)alkyl](lower)alkylcarbamoyloxy(lower) alkylcarbamoyl; lower alkylcarbamoyloxy(lower)alkenoyl; N-[heteroocyclic carbonyloxy(lower)alkyl](lower)alkylcarbamoyl; cyclo(lower)alkylcarbamoyl; arylcarbamoyl; haloarylcarbamoyl; protected carbamoyl; lower alkylthiocarbamoyl; heterocyclic carbamoyl; ar(lower)alkenoyl; lower alkoxycarbonyl; heterocyclic carbonyl which may contain lower alkyl, hydroxy, hydroxy(lower) alkyl, lower alkoxy(lower)alkyl or lower alkoxycarbonyl; lower alkyl, carboxy(lower)alkyl; protected carboxy(lower)alkyl; ar(lower)alkyl which may contain halogen

or lower alkoxy; heterocyclic(lower)alkyl; lower alkylcarbamoyl(lower)alkyl; hydroxy(lower)alkenoyl; acyloxy(lower)alkenoyl or diacyloxy(lower)alkenoyl;
$R^2$ is lower alkoxy; and
$R^3$ is represented by the following formula:

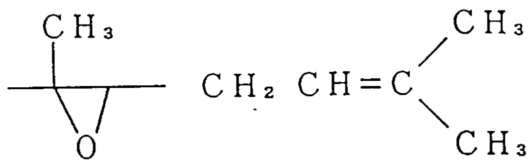

the following general formula:

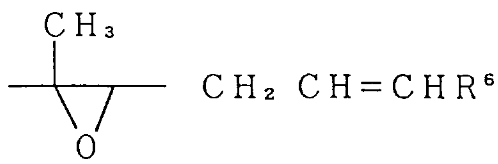

(wherein $R^6$ is protected carboxy),
the following general formula:

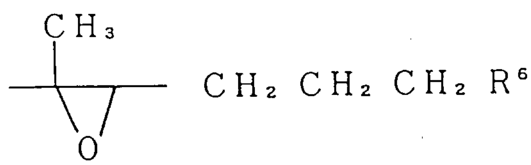

(wherein $R^6$ is the same as defined above),
the following formula:

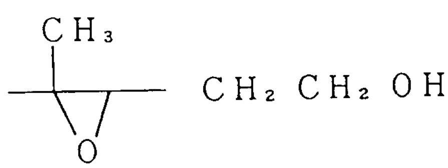

the following general formula:

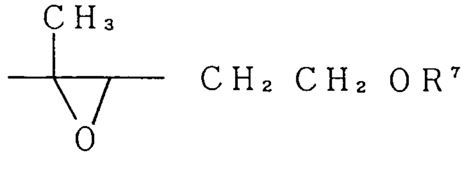

(wherein $R^7$ is protected carboxy(lower)alkyl, or ar(lower)alkyl which may contain halogen), or the following general formula:

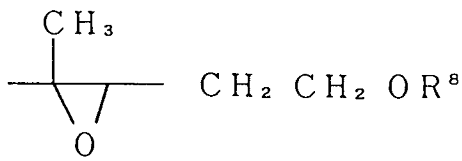

(wherein $R^8$ is acyl) or salts thereof, and pharmaceutically acceptable carrier(s).

**2.** The ophthalmic pharmaceutical preparation according to Claim 1, wherein
$R^1$ is lower alkylcarbamoyl,
$R^2$ is lower alkoxy, and
$R^3$ is represented by the following formula:

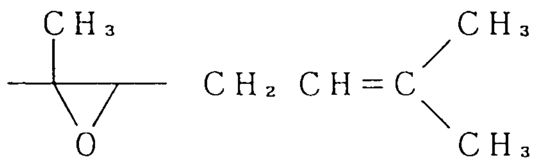

**3.** The ophthalmic pharmaceutical preparation according to Claim 2, wherein
$R^1$ is methylcarbamoyl,
$R^2$ is methoxy and
$R^3$ is the same as defined in Claim 2.

**4.** The ophthalmic pharmaceutical preparation according to any claim of Claims 1 to 3, wherein the oxaspirooctane derivatives (I) or salts thereof are mixed with a hydrocarbon gel, thereafter melted and then cooled rapidly for separating the oxaspirooctane derivatives (I) or salts thereof as microcrystalline to be ointment of a suspension-type.

**5.** The ophthalmic pharmaceutical preparation according to Claim 4, wherein the hydrocarbon gel is a mixture of liquid paraffin and polyethylene resin.

**6.** A treatment method for diseases caused by vascularization, wherein the ophthalmic pharmaceutical preparations according to any claim of Claims 1 to 5 are administered through eye to a patient with the diseases.

# INTERNATIONAL SEARCH REPORT

International Application No PCT/JP92/01177

**I. CLASSIFICATION OF SUBJECT MATTER** (if several classification symbols apply, indicate all) [6]

According to International Patent Classification (IPC) or to both National Classification and IPC

Int. $Cl^5$ A61K31/335, A61K9/08

**II. FIELDS SEARCHED**

Minimum Documentation Searched [7]

| Classification System | Classification Symbols |
|---|---|
| IPC | A61K31/335, A61K9/08 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | JP, A, 2-85272 (Fujisawa Pharmaceutical Co., Ltd.), March 26, 1990 (26. 03. 90), Claim and page 17 & EP, A, 354787 & US, A, 4954496 | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 30, 1992 (30. 11. 92) | December 22, 1992 (22. 12. 92) |
| **International Searching Authority** | **Signature of Authorized Officer** |
| Japanese Patent Office | |

International Application No. PCT/JP92/01177

**FURTHER INFORMATION CONTINUED FROM THE SECOND SHEET**

**V. [X] OBSERVATIONS WHERE CERTAIN CLAIMS WERE FOUND UNSEARCHABLE [1]**

This international search report has not been established in respect of certain claims under Article 17(2) (a) for the following reasons:

1. [X] Claim numbers 6 , because they relate to subject matter not required to be searched by this Authority, namely:

Claim 6 pertains to a medical treatment of the human body by curing.

2. [ ] Claim numbers , because they relate to parts of the international application that do not comply with the prescribed requirements to such an extent that no meaningful international search can be carried out, specifically:

3. [ ] Claim numbers , because they are dependent claims and are not drafted in accordance with the second and third sentences of PCT Rule 6.4(a).

**VI. [ ] OBSERVATIONS WHERE UNITY OF INVENTION IS LACKING [2]**

This International Searching Authority found multiple inventions in this international application as follows:

1. [ ] As all required additional search fees were timely paid by the applicant, this international search report covers all searchable claims of the international application.
2. [ ] As only some of the required additional search fees were timely paid by the applicant, this international search report covers only those claims of the international application for which fees were paid, specifically claims:
3. [ ] No required additional search fees were timely paid by the applicant. Consequently, this international search report is restricted to the invention first mentioned in the claims; it is covered by claim numbers:
4. [ ] As all searchable claims could be searched without effort justifying an additional fee, the International Searching Authority did not invite payment of any additional fee.

**Remark on Protest**

- [ ] The additional search fees were accompanied by applicant's protest.
- [ ] No protest accompanied the payment of additional search fees.